# EUROPÄISCHE PATENTSCHRIFT

(11) **EP 2 491 895 B1**
(45) Veröffentlichungstag und Bekanntmachung des Hinweises auf die Patenterteilung: **11.11.2015**
(21) Anmeldenummer: 11001626.8
(22) Anmeldetag: 28.02.2011
(51) Int. Cl.: A61F 5/01, A61F 13/06

(54) **Sprunggelenksorthese**
Ankle joint orthosis
Orthèse de cheville

(43) Veröffentlichungstag der Anmeldung: 29.08.2012
(73) Patentinhaber: BSN medical GmbH, 20253 Hamburg (DE)
(72) Erfinder: Grunden, Jennifer, 22769 Hamburg (DE); Scheib, Birgit, 22529 Hamburg (DE)
(74) Vertreter: UEXKÜLL & STOLBERG

(56) Entgegenhaltungen:
- US-A- 5 067 486
- US-A- 5 795 316
- US-A1- 2007 049 857
- US-A1- 2009 105 704
- US-B1- 7 753 865

## Beschreibung

Die vorliegende Erfindung betrifft eine Sprunggelenksorthese mit einem flexiblen Hauptkörper, der ausgebildet ist, um ein menschliches Bein im Bereich des Sprunggelenks vom Unterschenkel bis zum Mittelfuß zu umgeben, wobei der Hauptkörper einen medialen Abschnitt, einen lateralen Abschnitt, einen Fußsohlenabschnitt mit einer lateralen und einer medialen Seite, und einen Spann- und Ristabschnitt umfasst, wobei der mediale Abschnitt zur Anlage an die mediale Seite, der laterale Abschnitt zur Anlage an die laterale Seite, der Fußsohlenabschnitt zur Anlage an die Fußsohle und der Spann- und Ristabschnitt zur Anlage an den Spann und/oder den Rist des Fußes vorgesehen ist, und mit einem um den Hauptkörper wickelbaren Fixiergurt, der ein erstes Ende und ein zweites Ende aufweist, wobei der Fixiergurt mit dem ersten Ende mit dem lateralen Abschnitt des Hauptkörpers verbunden ist und mit dem zweiten Ende mit dem medialen Abschnitt des Hauptkörpers benachbart zu dessen oberem Ende verbunden ist und wobei der Fixiergurt derart verschiebbar um den Hauptkörper geführt ist, dass der Fixiergurt von dem ersten Ende an dem lateralen Abschnitt ausgehend über den Spann- und Ristabschnitt, zur medialen Seite des Fußsohlenabschnitts, über den Fußsohlenabschnitt hinweg zur lateralen Seite des Fußsohlenabschnitts, über den Spann- und Ristabschnitt und bis zum oberen Ende des medialen Abschnitts verläuft, wobei der Fixiergurt um den Fußsohlenabschnitt herum verläuft, ohne mit dem Hauptkörper verbunden zu sein, sondern an diesem anliegt und verschiebbar dazu ist, wobei eines aus dem ersten und dem zweiten Ende mit dem Hauptkörper in einer relativ zu dem Hauptkörper fest vorgegebenen Position fest oder lösbar verbunden ist und wobei das andere aus dem ersten und dem zweiten Ende lösbar mit dem Hauptkörper verbunden ist. Sprunggelenksorthesen werden auf dem Gebiet der orthopädischen Therapie zum Stabilisieren und zum Korrigieren der Stellung des Fußes relativ zum Unterschenkel verwendet. Zum Korrigieren dieser Stellung erfolgt häufig eine Anhebung des lateralen Abschnitts (Pronation) oder des medialen Abschnitts (Supination) des Fußes. Dabei können Sprunggelenksorthesen z.B. zur Behandlung von Bänderrissen oder -dehnungen im Sprunggelenk eingesetzt werden.

Aus dem Stand der Technik, wie beispielsweise der U.S. 2007/0049857, von der die vorliegende Erfindung ausgeht, ist eine Sprunggelenksorthese bekannt, bei der ein Fixiergurt fest mit dem Fußsohlenabschnitt des Hauptkörpers verbunden ist. Ferner ist der Fixiergurt mit seinem ersten Ende lösbar mit dem lateralen Abschnitt verbunden und mit seinem zweiten Ende lösbar mit dem medialen Abschnitt des Hauptkörpers benachbart zu dessen oberem Ende verbunden. Die lösbaren Verbindungen erfolgen jeweils über einen Haken-und-Flausch-Verschluss.

Eine lösbare Haken-und-Flausch-Verbindung gibt jedoch stets etwas nach, wenn der Fixiergurt vorgespannt wird, z.B. um eine Pronation oder eine Supination einzustellen. Ein genaues Maß an Pronation oder Supination kann nicht dauerhaft eingestellt werden, wenn das Ende des Fixiergurtes, welches dem Ende gegenüber liegt, an dem die Vorspannung eingeleitet wird, nicht starr sondern nachgiebig mit dem Hauptkörper verbunden ist und die Verbindung somit keine konstante Gegenkraft aufnehmen kann. Eine solche Verbindung an beiden Enden des Fixiergurts erweist sich somit als nachteilhaft.

Außerdem kann der Fixiergurt vollständig von dem Hauptkörper gelöst werden, was dem Benutzer beim nächsten Anlegen der Sprunggelenksorthese unnötige Schwierigkeiten oder Mühe bereiten kann.

Dadurch, dass der Fixiergurt fest mit dem Fußsohlenabschnitt des Hauptkörpers verbunden ist, ergibt sich zum einen der Nachteil, dass der Fixiergurt nicht mehr frei entlang des Fußsohlenabschnitts verschoben werden kann, um eine individuell optimale Lage zu suchen. Zum anderen führt eine feste Verbindung des Fixiergurts mit dem Fußsohlenabschnitt - wenn an dem ersten und zweiten Ende keine gleich große Vorspannung aufgebracht wird - zu einer Verdrehung bzw. Verschiebung des Hauptkörpers relativ zu einem von dem Hauptkörper umgebenen Fuß. Eine Pronation oder Supination kann nicht effektiv eingestellt werden. Ferner muss der Benutzer die Vorspannung an zwei Enden des Fixiergurts einstellen und evtl. auch aufeinander abstimmen, was das Anlegen der Sprunggelenksorthese verkompliziert.

Aus all dem ergibt sich, dass mit der Orthese gemäß der U.S. 2007/0049857 das Fußgelenk zwar fixiert werden kann, eine Korrektur der Stellung der Knochen relativ zueinander aber nicht möglich ist.

Aus der U.S. 5,067,486 ist außerdem eine Sprunggelenksorthese bekannt, bei der zwei Fixiergurte im Fersenbereich eines Hauptkörpers befestigt sind, wobei der erste Gurt zunächst auf dem lateralen Abschnitt des Hauptkörpers entlang läuft, dann über den Spann zu der medialen Seite und unter dem Fußsohlenabschnitt verläuft und schließlich wieder an der lateralen Seite lösbar befestigt ist. Der zweite Gurt läuft über den medialen Abschnitt, dann über den Spann zur lateralen Seiten und schließlich unter dem Fußsohlenabschnitt zurück zur medialen Seite, auf der er ebenfalls lösbar befestigt werden kann.

Auch mit dieser Orthese lässt sich die Stellung der Knochen im Bereich des Fußgelenks nicht korrigieren, sondern das Fußgelenk kann ebenfalls nur fixiert werden. Eine Einstellung einer Pronation oder Supination ist aufgrund der Gurtführung auch hier nicht möglich.

Ausgehend vom Stand der Technik ist es daher die Aufgabe der vorliegenden Erfindung, eine Sprunggelenksorthese bereitzustellen, die es erlaubt, auf besonders einfache Weise eine Pronation oder Supination auf ein genaues Maß einzustellen und dabei möglichst bequem tragbar zu sein.

Diese Aufgabe wird dadurch gelöst, dass an dem lateralen Abschnitt des Hauptkörpers ein laterales Schienenelement und an dem medialen Abschnitt des Hauptkörpers ein mediales Schienenelement angebracht ist.

Falls die Sprunggelenksorthese zur Einstellung einer Pronation eingesetzt werden soll, ist der Fixiergurt mit seinem ersten Ende fest mit dem lateralen Abschnitt des Hauptkörpers verbunden und mit seinem zweiten Ende lösbar mit dem medialen Abschnitt des Hauptkörpers verbunden. Die lösbare Verbindung kann z.B. über einen Haken-und-Flausch-Verschluss erfolgen.

Alternativ kann der Fixiergurt mit seinem ersten Ende mit dem Hauptkörper in einer relativ zu dem Hauptkörper fest vorgegebenen Position lösbar verbunden sein. Diese lösbare Verbindung in einer fest vorgegebenen Position kann z.B. mit Hilfe eines oder mehrerer Druckverschlüsse erfolgen. Druckverschlüsse stellen ein starres Verbindungselement dar, welches nicht nachgibt, wenn eine Vorspannung auf den Fixiergurt wirkt. Somit kann auch mit dieser Verbindung ein genaues Maß an Pronation oder Supination eingestellt werden.

In dem Fall, dass die Sprunggelenksorthese zur Einstellung einer Supination eingesetzt werden soll, ist der Fixiergurt mit seinem zweiten Ende fest oder alternativ in einer relativ zu dem Hauptkörper fest vorgegebenen Position lösbar, z.B. über einen Druckknopfverschluss, mit dem medialen Abschnitt des Hauptkörpers verbunden und mit seinem ersten Ende lösbar, z.B. über einen Haken-und-Flausch-Verschluss, mit dem lateralen Abschnitt des Hauptkörpers verbunden.

Der Fixiergurt ist nur in dem Bereich mit dem Hauptkörper verbunden, der benachbart zu dem oberen Ende des Hauptkörpers ist. Um den Fußsohlenabschnitt herum verläuft der Fixiergurt, ohne mit dem Hauptkörper verbunden zu sein, sondern liegt lediglich an diesem an und ist verschiebbar dazu. Somit kann der Fixiergurt beim Einstellen des Maßes an Pronation bzw. Supination gleitend entlang des Fußsohlenabschnitts bewegt werden, ohne den Hauptkörper der Orthese zu verdrehen.

Wenn ein Benutzer beispielsweise eine Pronation einstellen möchte und dazu den erfindungsgemäßen Fixiergurt an seinem zweiten Ende auf Spannung bringt, wird der Hauptkörper mit dem von dem Hauptkörper umgebenen Fuß zu der lateralen Seite des Fußsohlenabschnitts hin zunehmend angehoben. Es erfolgt eine gleichmäßige und effektive Pronation des Fußes, wobei das gewünschte Maß an Pronation durch den entsprechenden Zug an dem zweiten Ende des Fixiergurtes eingestellt werden kann.

Außerdem ist der Fixiergurt vorzugsweise aus einem in Längsrichtung des Gurts im Wesentlichen inelastischen Material ausgebildet, um eine Dehnung des Gurts zu vermeiden, sodass das Maß der Pronation bzw. Supination auch dauerhaft eingestellt bleibt.

Zur Anbringung des Fixiergurtes und damit Fixierung eines bestimmten Maßes an Pronation, kann das zweite Ende des Fixiergurtes mit Hilfe eines Haken-und-Flausch-Verschlusses, vorzugsweise eines Klettverschlusses, lösbar mit dem Bereich des medialen Abschnitts des Hauptkörpers verbunden werden, der an das obere Ende des Hauptkörpers angrenzt. Um verschiedene Einstellungsstufen wählen und so verschiedene Maße an Pronation einstellen zu können, kann der Haken-und-Flausch-Verschluss so breit ausgeführt sein, dass das zweite Ende des Fixiergurts an mehreren in Erstreckungsrichtung des Fixiergurtes voneinander beabstandeten Orten mit dem Hauptkörper verbunden werden kann.

Der Fixiergurt liegt vorzugsweise in im Wesentlichen symmetrischer Anordnung schlaufenartig um den Hauptkörper herum an diesem an, so dass der Fixiergurt den Fußsohlenabschnitt im Bereich des Mittelfußes umschließt, sich dann im Bereich des Spann- und Ristabschnitts kreuzt, um schließlich mit seinen beiden Enden an dem Bereich des lateralen bzw. medialen Abschnitt des Hauptkörpers angebracht zu sein, der benachbart zu dem oberen Ende des Hauptkörpers ist.

In einer bevorzugten Ausführungsform ist das eine Ende fest mit dem Hauptkörper verbunden. Auf diese Weise kann ein genaues Maß an Pronation eingestellt werden. Außerdem erleichtert dies dem Benutzer das Anlegen des Fixiergurtes.

In einer anderen bevorzugten Ausführungsform ist das eine Ende in einer aus einer Vielzahl von relativ zu dem Hauptkörper fest vorgegebenen Positionen lösbar mit diesem verbunden. Dabei ist es besonders bevorzugt, wenn das eine Ende über eine oder mehrere Druckknopfverbindung mit dem Hauptkörper verbunden ist. Damit kann zum einen ein genaues Maß an Pronation eingestellt werden, zum anderen der Fixiergurt aber auch vollständig von der Sprunggelenksorthese entfernt werden, beispielsweise um ausgetauscht zu werden. Gleichzeitig kann die Position der starren Verbindung aber auch an individuelle Bedürfnisse der Benutzer angepasst werden. Druckknöpfe stellen ein geeignetes lösbares jedoch auch starres Verbindungselement dar.

In einer weiteren bevorzugten Ausführungsform ist das andere Ende über eine Haken-und-Flausch-Verbindung lösbar mit dem Hauptkörper verbunden. Eine Haken-und-Flausch-Verbindung ist schnell und leichtgängig zu öffnen und zu verschließen und ermöglicht eine Fixierung an einer in einem bestimmten Bereich kontinuierlich veränderbaren Position. Ein genaues, individuell gewünschtes Maß an Pronation bzw. Supination kann auf diese Weise fixiert werden.

Dadurch, dass das eine Ende fest oder in einer fest vorgegebenen Position lösbar und das andere Ende lösbar mit dem Hauptkörper verbunden ist, wird für den Benutzer außerdem unmittelbar klar, in welcher Reihenfolge die Enden an dem Hauptkörper angebracht werden müssen, wie also der Fixiergurt gespannt werden muss. Dadurch wird sichergestellt, dass der Benutzer tatsächlich die Pronation bzw. Supination wie vorgegeben einstellt und den Fixiergurt nicht etwa in die falsche Richtung spannt.

In noch einer weiteren bevorzugten Ausführungsform weist der Hauptkörper entlang des Verlaufs des Fixiergurts Führungslaschen zur verschiebbaren Führung des Fixiergurts auf. Mit solchen Führungslaschen kann der Fixiergurt beweglich entlang seiner gewünschten Verlaufsrichtung fixiert werden. Die Führungslaschen dienen dazu, die Benutzung der Sprunggelenksorthese zu vereinfachen und eine präzise und reproduzierbare Anordnung des Fixiergurts zu gewährleisten, und sind vorzugsweise im Bereich des Spann- und Ristabschnitts an dem Hauptkörper vorgesehen.

An dem lateralen Abschnitt des Hauptkörpers ist erfindungsgemäß ein laterales Schienenelement und an dem medialen Abschnitt des Hauptkörpers ein mediales Schienenelement angebracht. Die Schienenelemente dienen zur Schienung, d.h. einer grundsätzlichen Versteifung des Bereichs zwischen Unterschenkel und Sprunggelenk. Sie liegen dabei von gegenüberliegenden Seiten an dem Unterschenkel an.

In einer anderen bevorzugten Ausführungsform ist an dem Hauptkörpers eine Verstärkungsapplikation vorgesehen, die zusammenhängend ausgebildet ist und sich von dem oberen Ende des lateralen Abschnitts über diesen, über den Fußsohlenabschnitt und über den medialen Abschnitt bis zu dessen oberem Ende erstreckt. Die Verstärkungsapplikation kann zum Halten der Schienenelemente an einer geeigneten Position dienen. Ein weiterer Effekt der Verstärkungsapplikation ist, dass sie den Fußsohlenabschnitt verstärkt und das Zusammenhalten des lateralen und des medialen Abschnitts gewährleistet.

Dabei ist es besonders bevorzugt, wenn das Material der Verstärkungsapplikation in dessen Verlaufsrichtung vom oberen Ende des lateralen Abschnitts zum oberen Ende des medialen Abschnitts nicht-dehnbar ausgebildet ist. Ein in eine Richtung nicht-dehnbares, vorzugsweise anisotropes Material, z.B. ein faserverstärkter Kunststoff kann für die Verstärkungsapplikation verwendet werden, um ein Aufweiten des Fußsohlenabschnitts in Richtung zwischen seiner lateralen und medialen Seite zu verhindern und somit die Form der Sprunggelenksorthese zu erhalten.Besonders bevorzugt ist es auch, wenn das laterale und das mediale Schienenelement zwischen dem Hauptkörper und einer Verstärkungsapplikation angebracht sind. Auf diese Weise kann die Verstärkungsapplikation die Schienenelemente an dem gewünschten Ort festhalten, ohne dass eine gesonderte Anbringung für die Schienenelemente nötig ist.

Gemäß einem bevorzugten Ausführungsbeispiel, das auch selbstständig erfinderisch ist, also auch allgemein bei einer Sprunggelenksorthese mit einem flexiblen Hauptkörper ohne die zuvor beschriebene Gurtführung Anwendung finden kann, weist der Hauptkörper einen posterioren Abschnitt auf, der vorgesehen ist, an der posterioren Seite des Fußes anzuliegen, einen lateralen Knöchelabschnitt auf, der vorgesehen ist, auf der lateralen Seite des Fußes an dem Knöchel anzuliegen und einen medialen Knöchelabschnitt auf, der vorgesehen ist, auf der medialen Seite des Fußes an dem Knöchel anzuliegen, wobei das laterale Schienenelement an dem lateralen Abschnitt des Hauptkörpers derart vom posterioren Abschnitt weg versetzt verläuft, dass das laterale Schienenelement gegenüber dem lateralen Knöchelabschnitt in Richtung zum Spann- und Ristabschnitt versetzt verläuft und wobei das mediale Schienenelement an dem medialen Abschnitt des Hauptkörpers derart zum posterioren Abschnitt hin versetzt angeordnet ist, dass das mediale Schienenelement gegenüber dem medialen Knöchelabschnitt in Richtung zum posterioren Abschnitt versetzt verläuft.

Dabei kann bei dem allgemein selbstständig erfinderischen Konzept der Hauptkörper der Sprunggelenksorthese so ausgebildet sein, dass ein Einstieg sowohl von hinten, also über eine Öffnung im posterioren Abschnitt, als auch von vorne über einen Einstiegsbereich im Spann- und Ristabschnitt erfolgen kann.

Durch eine derart gegenüber den Knöchelabschnitten versetzte Anordnung der Schienenelemente wird erreicht, dass durch die Orthese ein sogenannter Talusvorschub verhindert wird, also eine Verschiebung des Sprungbeins nach vorne in Bezug auf die Schienbeingelenkfläche. Es wird also durch die versetzte Lage der Schienenelemente eine Kraft auf das Sprungbein ausgeübt, wenn das Sprungbein sich in Bezug auf die Schienbeingelenkfläche nach vorne verschiebt.

Ein weitere Vorteil dieser Anordnung der Schienenelemente ist, dass sie dabei nicht unmittelbar am Knöchel anliegen, was leicht zu Schmerzen beim Benutzer führen kann, wenn ein Druck auf die Schienenelemente ausgeübt wird.

In einer weiteren bevorzugten Ausführungsform ist ein Kompressionsgurt aus einem elastischen Material vorgesehen, der ein erstes und ein zweites Ende aufweist, wobei der Kompressionsgurt um den Hauptkörper, den Fixiergurt und die Verstärkungsapplikation gewickelt ist, und wobei das erste Ende des Kompressionsgurts fest und das zweite Ende des Kompressionsgurts lösbar mit dem Hauptkörper, dem Fixiergurt oder der Verstärkungsapplikation verbunden ist.

Mit einem Kompressionsgurt kann zusätzlich zu einer Korrektur (Pronation bzw. Supination) durch den Fixiergurt und ggf. einer Schienung durch die Schienenelemente eine Kompression der unteren Extremitäten im Bereich des Sprunggelenks erzielt werden. Der Kompressionsgurt hat somit eine ähnliche Wirkung wie die einer Bandage. Außerdem sorgt der Kompressionsgurt dafür, dass die darunter angeordneten Schienenelemente möglichst eng und starr an den Unterschenkelknochen, -sehnen und -bändern anliegen und eine korrigierende Funktion ausüben. Das gewünschte Maß an Kompression kann durch ein Spannen des Kompressionsgurts eingestellt werden.

Dabei ist es besonders bevorzugt, wenn das erste Ende des Kompressionsgurts fest an dem posterioren Abschnitt angebracht ist, wenn der Kompressionsgurt ausgebildet ist, vom posterioren Abschnitt über den lateralen Abschnitt zur lateralen Seite des Fußsohlenabschnitts, über den Fußsohlenabschnitt zu dessen medialer Seite, über den medialen Abschnitt und den Spann- und Ristabschnitt zum lateralen Abschnitt, über den lateralen Abschnitt, den posterioren Abschnitt und über den medialen Abschnitt nach oben zu dem Bereich des lateralen Abschnitts zu verlaufen, der benachbart zum oberen Ende des lateralen Abschnitts ist, und wenn das zweite Ende des Kompressionsgurts in dem Bereich des lateralen Abschnitts lösbar verbunden ist, der benachbart zum oberen Ende des lateralen Abschnitts ist.

Durch diese Führung des Kompressionsgurts entlang von Hauptkörper, Verstärkungsapplikation und Fixiergurt wird der Druck, der durch ein Spannen des Kompressionsgurtes aufgebracht wird, in möglichst gleichmäßiger Weise auf die darunter liegende Struktur (Hauptkörper, Verstärkungsapplikation und Fixiergurt) verteilt. Es treten keine unangenehmen oder orthopädisch nachteiligen Druckspitzen auf.

Das zweite Ende des Kompressionsgurts kann über einen Haken-und-Flausch-Verschluss, bzw. über einen Klettverschluss, lösbar mit dem lateralen Abschnitt verbunden sein. Zur lösbaren Anbringung des Zweiten Endes des Kompressionsgurts kann in dem Bereich des lateralen Abschnitts, der benachbart zum oberen Ende des Hauptkörpers ist, vorzugsweise auf der Verstärkungsapplikation eine Haken-und-Flausch-Verschlussunterlage vorgesehen sein, die so dimensioniert ist, dass der Kompressionsgurt mit seinem zweiten Ende an verschiedenen in Erstreckungsrichtung des Kompressionsgurtes voneinander beabstandeten Punkten an der Haken-und-Flausch-Verschlussunterlage angebracht werden kann, um das eingestellte Maß an Kompression auf diese Weise zu fixieren.

Ferner ist es besonders bevorzugt, wenn der Kompressionsgurt an dem Fußsohlenabschnitt fixiert ist. Eine solche Fixierung des Kompressionsgurtes, die vorzugsweise im Bereich der Ferse eines von dem Hauptkörper umgebenen Fußes vorgesehen ist, wird die Führung des Kompressionsgurtes beim Anlegen der Sprunggelenksorthese erleichtert, bzw. wird ein reproduzierbarer Ort für die Führung im Bereich des Fußsohlenabschnitts gewährleistet.

Die vorliegende Erfindung wird im Folgenden anhand einer ein Ausführungsbeispiel darstellenden Zeichnung erläutert. Die Zeichnung zeigt in
- Fig. 1: eine anteriore Ansicht eines Ausführungsbeispiels einer Sprunggelenksorthese,
- Fig. 2: eine laterale Ansicht des Ausführungsbeispiels aus Fig. 1,
- Fig. 3: eine mediale Ansicht des Ausführungsbeispiels aus Fig. 1,
- Fig. 4: eine posteriore Ansicht des Ausführungsbeispiels aus Fig. 1,
- Fig. 5: eine plantare Ansicht des Ausführungsbeispiels aus Fig. 1,
- Fig. 6: eine zweite anteriore Ansicht des Ausführungsbeispiels aus Fig. 1, wobei der Kompressionsgurt entfernt ist, und
- Fig. 7: vier perspektivische Ansichten der Lage der Schienenelemente eines Ausführungsbeispiels einer Sprunggelenksorthese relativ zu einem Fuß, welcher von der Sprunggelenksorthese umgeben werden kann.

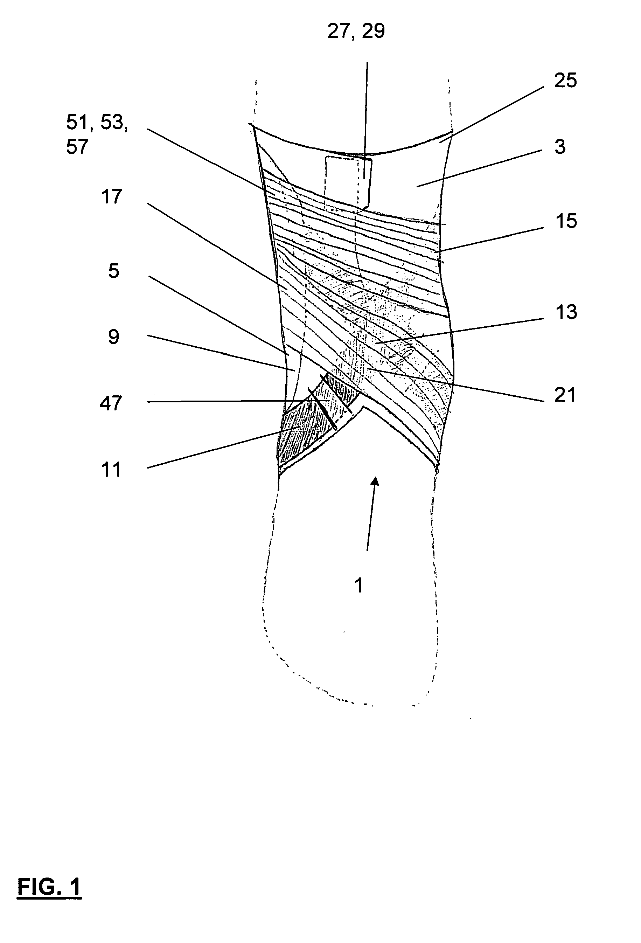

In Fig. 1 ist ein Ausführungsbeispiel einer Sprunggelenksorthese 1 dargestellt, die im vorliegenden Fall zur Einstellung der Pronation eines von ihr umgebenen Fußgelenks eingesetzt werden kann. Die Sprunggelenksorthese 1 umfasst einen Hauptkörper 3, ein laterales und ein mediales Schienenelement 5, 7, eine Verstärkungsapplikation 9, einen Fixiergurt 11 und einen Kompressionsgurt 13.

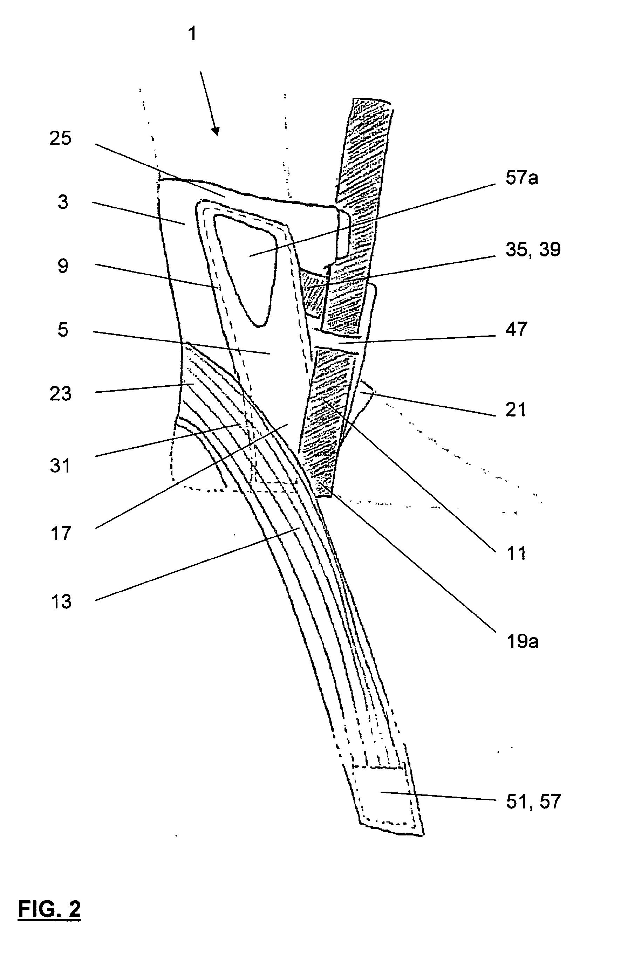

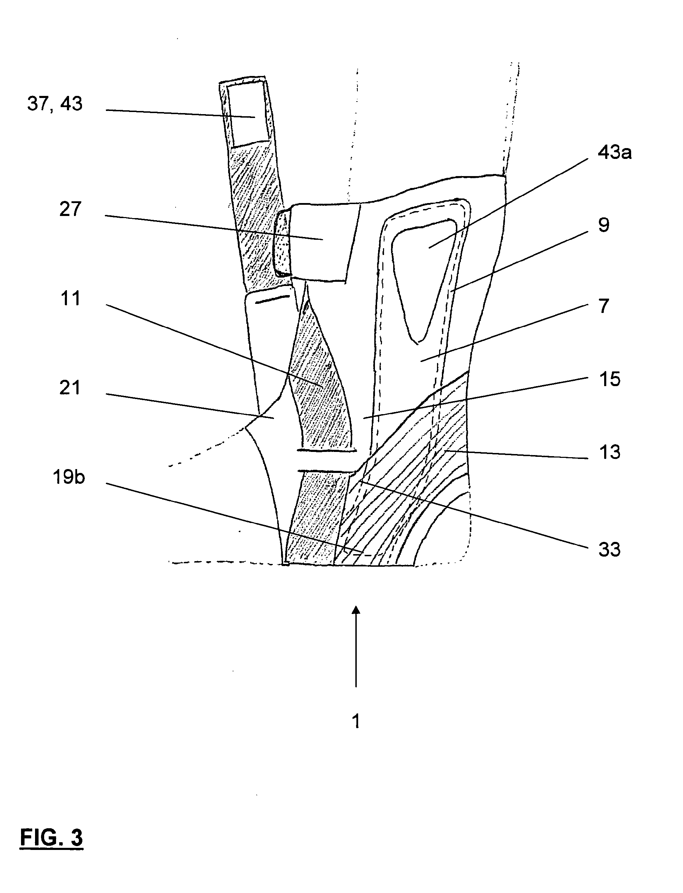

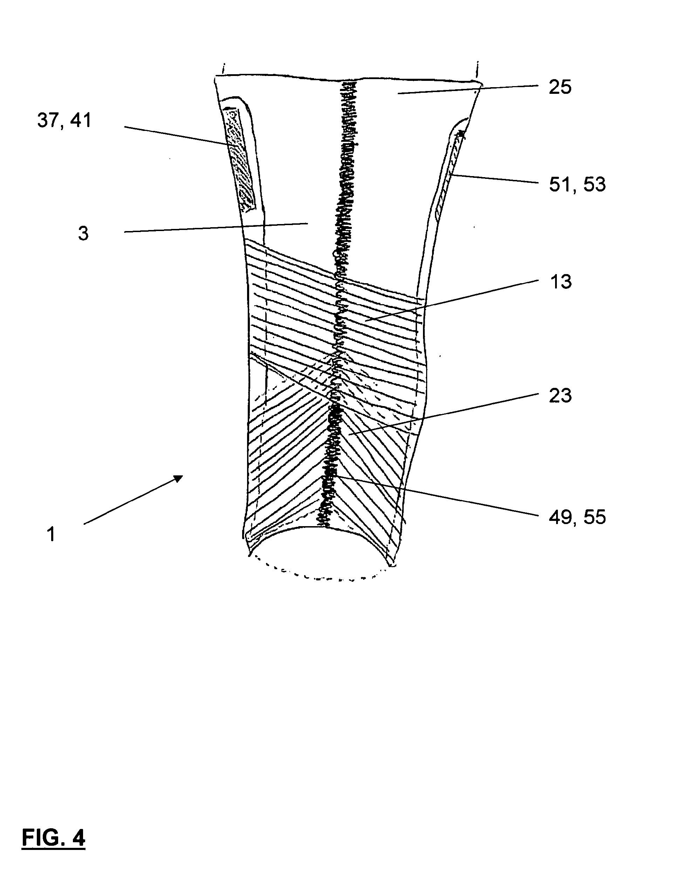

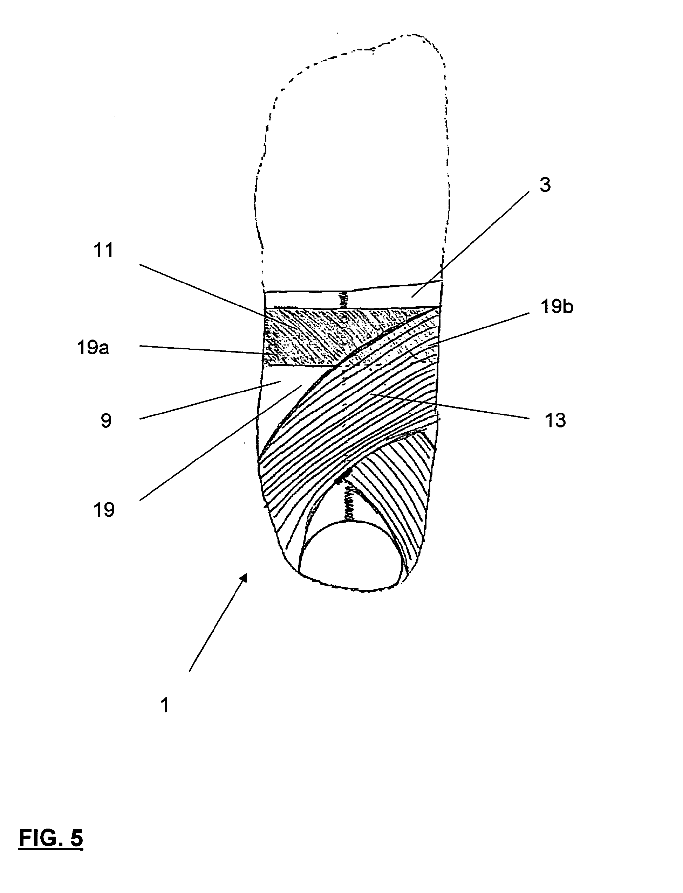

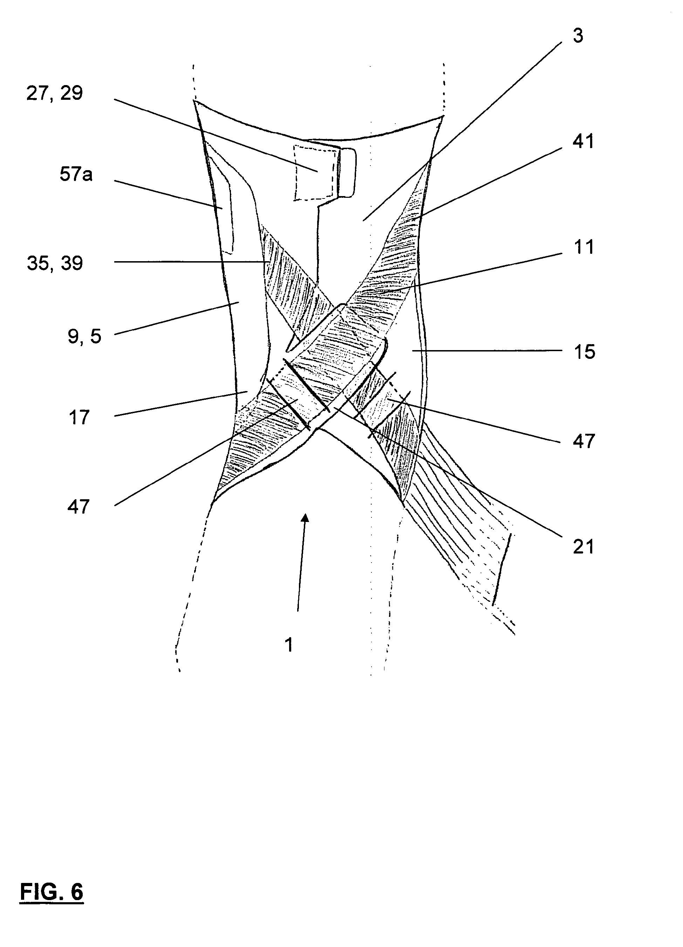

Der Hauptkörper 3 ist aus flexiblem Material derart ausgebildet, um ein menschliches Bein im Bereich des Sprunggelenks vom Unterschenkel bis zum Mittelfuß zu umgeben, d.h. eng an diesem anzuliegen. Dabei weist der Hauptkörper 3 einen medialen Abschnitt 15 (siehe Fig. 3), einen lateralen Abschnitt 17 (siehe Fig. 2), einen Fußsohlenabschnitt 19 (siehe Fig. 5) mit einer lateralen und einer medialen Seite 19a, 19b, einen Spann- und Ristabschnitt 21 (siehe Fig. 1 und 6) und einen posterioren Abschnitt 23 (siehe Fig. 4) auf. Die Abschnitte können einstückig miteinander ausgebildet sein, oder der Hauptkörper 3 kann miteinander beispielsweise vernähte Abschnitte aufweisen.

Der mediale Abschnitt 15 ist zur Anlage an die mediale Seite, der laterale Abschnitt 17 zur Anlage an die laterale Seite, der Fußsohlenabschnitt 19 zur Anlage an die Fußsohle, der Spann- und Ristabschnitt 21 zur Anlage an den Spann und/oder den Rist und der posteriore Abschnitt 23 zur Anlage an die posteriore Seite eines von dem Hauptkörper 3 umgebenen Fußes bzw. eines Beins im Bereich des Sprunggelenks vom Unterschenkel bis zum Mittelfuß vorgesehen.

Der Hauptkörper 3 weist ein oberes Ende 25 auf, das den einen Fuß umgebenden Hauptkörper 3 auf Seiten des Unterschenkels, d. h. entfernt vom Fußsohlenabschnitt 19 gelegen, begrenzt.

Der Hauptkörper 3 weist ferner einen Öffnungsabschnitt 27 auf, der sich vom Spann- und Ristabschnitt 21 hin zum oberen Ende 25 des Hauptkörpers 3 erstreckt. Der Öffnungsabschnitt 27 ist durch eine Lasche 29 gebildet, die fest mit dem lateralen Abschnitt 17 des Hauptkörpers 3 verbunden bzw. einteilig mit diesem ausgebildet ist.

Zum Verschließen des Öffnungsabschnitts 27 kann die Lasche 29 in Anlage mit dem medialen Abschnitt 15 des Hauptkörpers 3 gebracht und lösbar mit diesem verbunden werden. Zum Öffnen des Öffnungsabschnitts 27 kann die Lasche 29 an dem medialen Abschnitt 15 des Hauptkörpers 3 gelöst werden und dann der mediale und der laterale Abschnitt 15, 17 voneinander entfernt werden, so dass der Hauptkörper 3 aufgeweitet wird. Auf diese Weise kann die Sprunggelenksorthese 1 leicht an einem Fuß angelegt werden.

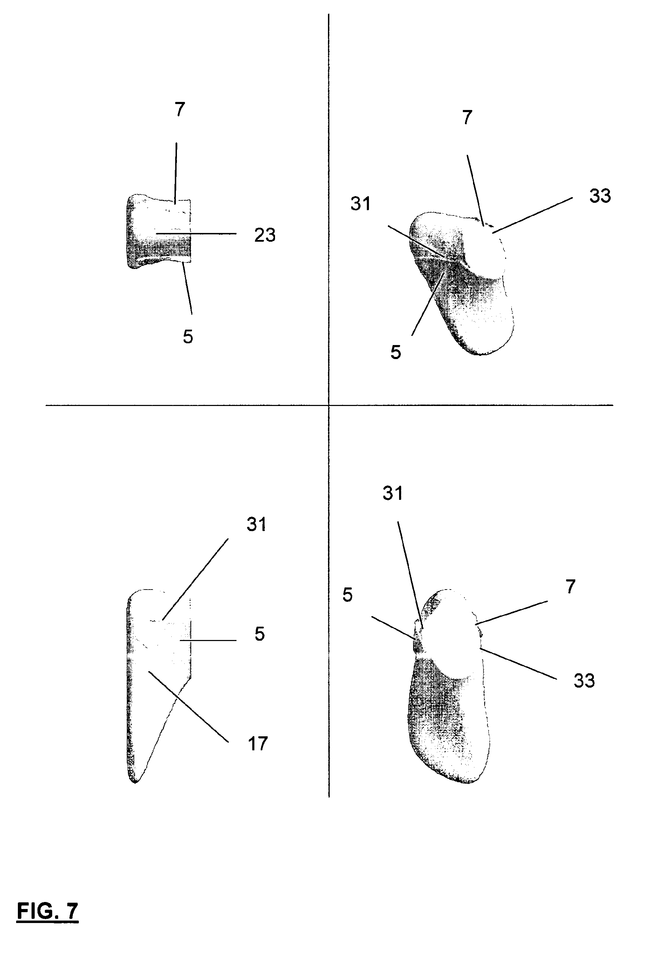

Als Alternative ist es allerdings auch denkbar, dass die Lasche 29 mit dem medialen Abschnitt 15 fest verbunden ist und lösbar mit dem lateralen Abschnitt 17 verbunden werden kann. Das laterale und das mediale Schienenelement 5, 7 sind mit Hilfe der Verstärkungsapplikation 9 an dem lateralen bzw. medialen Abschnitt 17, 15 des Hauptkörpers 3 angebracht. Dabei sind die Schienenelemente 5, 7 zwischen dem Hauptkörper 3 und der Verstärkungsapplikation 9 angeordnet und werden von der Verstärkungsapplikation 9 an dem Hauptkörper 3 gehaltert. Die Schienenelemente 5, 7 sind aus einem steifen Material ausgebildet und erstrecken sich von der lateralen bzw. medialen Seite 19a, 19b des Fußsohlenabschnitts 19 bis in den Bereich des lateralen bzw. medialen Abschnitts 17, 15, der an das obere Ende 25 des Hauptkörpers 3 angrenzt. Dabei ist das laterale Schienenelement 5 derart an dem lateralen Abschnitt 17 des Hauptkörpers 3 angeordnet, dass es gegenüber einem lateralen Knöchelabschnitt 31 in Richtung vom posterioren Abschnitt 23 des Hauptkörpers 3 weg versetzt verläuft (siehe Fig. 7). Gleichzeitig ist das mediale Schienenelement 7 derart an dem medialen Abschnitt 15 des Hauptkörpers 3 angebracht, dass es gegenüber dem medialen Knöchelabschnitt 33 in Richtung zum posterioren Abschnitt 23 des Hauptkörpers 3 hin versetzt verläuft. Der laterale Knöchelabschnitt 31 des Hauptkörpers 3 ist vorgesehen, auf der lateralen Seite des Fußes an dem Knöchel anzuliegen. Außerdem ist der medialen Knöchelabschnitt 33 vorgesehen, um auf der medialen Seite des Fußes an dem Knöchel anzuliegen. Diese Anordnung der Schienenelemente 5, 7 relativ zu dem Fuß eines Benutzers ist in Fig. 7 im Detail dargestellt, wobei dort durch die Bezugszeichen "17", "23", "31", "33" die Lage der dort nicht gezeigten Abschnitte des Hauptkörpers wiedergegeben ist.

Durch die gegenüber den Knöchelabschnitten jeweils versetzte Anordnung der Schienenelemente 5, 7 wird erreicht, dass ein sogenannter Talusvorschub verhindert wird. Einer Verschiebung des Sprungbeins nach vorne in Bezug auf die Schienbeingelenkfläche wird entgegengewirkt, wobei durch die versetzte Lage der Schienenelemente eine Kraft auf das Sprungbein ausgeübt wird, wenn das Sprungbein sich in Bezug auf die Schienbeingelenkfläche nach vorne verschiebt.

Durch diese Anordnung der Schienenelemente 5, 7 wird außerdem erreicht, dass sie nicht unmittelbar auf dem Knöchel des Benutzers anliegen, was sonst zu Schmerzen führen kann, wenn ein Druck auf die Schienenelemente 5, 7 ausgeübt wird.

Die Verstärkungsapplikation 9 ist zusammenhängend, also vorzugsweise einstückig ausgebildet, und erstreckt sich von dem Bereich des lateralen Abschnitts 17, der an das oberen Ende 25 des Hauptkörpers 3 angrenzt, entlang des lateralen Abschnitts 17 des Hauptkörpers 3 über das laterale Schienenelement 5 bis zur lateralen Seite 19a des Fußsohlenabschnitt 19, um den Fußsohlenabschnitt 19 herum bis zur medialen Seite 19b des Fußsohlenabschnitts 19 und entlang des medialen Abschnitts 15 des Hauptkörpers 3 über das mediale Schienenelement 7 bis zum Bereich des medialen Abschnitts 15, der an das obere Ende 25 des Hauptkörpers 3 angrenzt. In dieser Erstreckungsrichtung der Verstärkungsapplikation 9 vom oberen Ende 25 des lateralen Abschnitts 17 über den Fußsohlenabschnitt 19 bis zum oberen Ende 25 des medialen Abschnitts 15 des Hauptkörpers 3 ist die Verstärkungsapplikation 9 nicht-dehnbar ausgebildet.

Durch diesen nicht-dehnbaren Aufbau wird durch die Verstärkungsapplikation 9 verhindert, dass sich der Fußsohlenabschnitt 19 quer zu seiner Längserstreckung aufweiten kann, sodass sichergestellt ist, dass die Seiten 19a, 19b des Fußsohlenabschnitts 19 immer an einer definierten Position relativ zu dem Fuß des Benutzers angeordnet bleiben.

An dem Hauptkörper 3 ist ferner ein Fixiergurt 11 vorgesehen. Der Fixiergurt 11 ist aus einem Material mit hoher Festigkeit ausgebildet und weist ein erstes und ein zweites Ende 35, 37 auf. Vorzugsweise ist der Fixiergurt 11 aus einem inelastischen Material ausgebildet, sodass seine Länge in Erstreckungsrichtung im Wesentlichen unveränderlich ist. Das erste Ende 35 des Fixiergurts 11 ist im vorliegenden Ausführungsbeispiel fest mit dem Bereich des lateralen Abschnitts 17 des Hauptkörpers 3 verbunden, der benachbart zu dem oberen Ende 25 des Hauptkörpers 3 ist.

Es ist aber auch denkbar, dass das erste Ende 35 mit dem Hauptkörper 3 in einer relativ zu diesem fest vorgegebenen Position fest oder lösbar verbunden ist, beispielsweise durch eine Druckknopfverbindung. Dabei ist es auch denkbar, dass der Benutzer einen aus mehreren am Hauptkörper 3 vorgesehenen Druckknöpfen zur Befestigung des entsprechenden Gegenstücks am ersten Ende 35 des Fixiergurts 11 auswählen kann.

In jedem Fall ist die Position, an der das erste Ende 35 mit dem Hauptkörper 3 verbunden wird, festgelegt und nicht über einen Bereich verteilt, wie dies beispielsweise bei einem Haken-Flausch-Verschluss der Fall wäre.

Das zweite Ende 37 des Fixiergurts 11 ist lösbar mit dem Bereich des medialen Abschnitts 15 des Hauptkörpers 3 verbunden, der benachbart zu dem oberen Ende 25 des Hauptkörpers 3 ist.

Zwischen dem ersten und dem zweiten Ende 35, 37 erstreckt sich der Fixiergurt 11 schlaufenartig um den von dem Hauptkörper 3 umgebenen Fuß im Bereich des Mittelfußes herum und ist dabei gegenüber dem Hauptkörper 3 entlang seiner gesamten Länge verschiebbar. Der Fixiergurt 11 verläuft von seiner festen Anbringung 39 an dem Hauptkörper 3 mit seinem ersten Ende 35 im Bereich des lateralen Abschnitts 17 über den Spann- und Ristabschnitt 21 hin zur medialen Seite 19b des Fußsohlenabschnitts 19, um den Fußsohlenabschnitt 19 herum bis zur lateralen Seite 19a des Fußsohlenabschnitts 19 und von dort über den Spann- und Ristabschnitt 21 bis hin zu dem Ort der lösbaren Anbringung 41 des zweiten Endes 37 des Fixiergurts 11 an dem medialen Abschnitt 15 des Hauptkörpers 3.

Diese lösbare Verbindung des Fixiergurts 11 am Hauptkörper 3 ist in dem hier beschriebenen Ausführungsbeispiel durch einen Haken-und-Flausch-Verschluss 43, vorzugsweise durch einen Klettverschluss verwirklicht. Der Ort der lösbaren Verbindung 41 des Fixiergurts 11 an dem Hauptkörper 3 befindet sich in dem vorliegenden Ausführungsbeispiel auf der Verstärkungsapplikation 9 im medialen Abschnitt 15 des Hauptkörpers 3.

Um eine Pronation der lateralen Seite 19a des Fußsohlenabschnitts 19 zu erreichen, wird der Fixiergurt 11 auf Spannung gebracht, wobei er sich aufgrund der Verschiebbarkeit entlang seiner gesamten Länge relativ zu dem Hauptkörper 3 bewegen kann. Für eine flexible Anpassung des Maßes an Pronation, auf das der Fuß eingestellt werden soll, kann der Fixiergurt 11 - da der Haken-und-Flausch-Verschluss 43 an der Verstärkungsapplikation 9 eine gewisse Breite aufweist - in seiner Erstreckungsrichtung an unterschiedlich weit von der lateralen Seite 19a des Fußsohlenabschnitts 19 entfernten Orten lösbar angebracht werden. Der Ort der lösbaren Verbindung 41 des Fixiergurts 11 am Hauptkörper 3 ist somit wegen der großflächigen Gestalt der Haken-und-Flausch-Verschlussunterlage 43a in einem bestimmten an das oberen Ende 25 des Hauptkörpers 3 angrenzenden Bereich des medialen Abschnitts 15 flexibel anpassbar bzw. versetzbar.

Um den Fixiergurt 11 am Hauptkörper 3 zu führen bzw. beweglich entlang seiner Erstreckungsrichtung zu fixieren, sind Führungslaschen 47 vorgesehen, die im Bereich des Spann- und Ristabschnitts 21 am Hauptkörper 3 angebracht sind.

Zur zusätzlichen Kompression des Fußes ist ein weiterer Gurt - der Kompressionsgurt 13 - vorgesehen. Dieser Kompressionsgurt 13 weist ein elastisches Material und eine netzförmige Struktur auf. Der Kompressionsgurt 13 weist ferner ein erstes Ende 49 und ein zweites Ende 51 auf und kann um den Hauptkörper 3, den Fixiergurt 11 und die Verstärkungsapplikation 9 gewickelt werden, wobei das erste Ende 49 des Kompressionsgurts 13 lösbar an entweder dem Hauptkörper 3, dem Fixiergurt 11 oder an der Verstärkungsapplikation 9 angebracht ist. Im vorliegende Ausführungsbeispiel ist das erste Ende 49 des Kompressionsgurts 13 fest am posterioren Abschnitt 23 des Hauptkörpers 3 angebracht, und das zweite Ende 51 des Kompressionsgurts 13 ist lösbar in dem Bereich des lateralen Abschnitts 17 des Hauptkörpers 3 mit der Verstärkungsapplikation 9 verbunden, der benachbart ist zu dem oberen Ende 25 des Hauptkörpers 3.

Auch diese lösbare Verbindung 53 des Kompressionsgurts 13 an der Verstärkungsapplikation 9 im lateralen Abschnitt 17 des Hauptkörpers 3 ist durch einen Haken-und-Flausch-Verschluss 57, vorzugsweise durch einen Klettverschluss verwirklicht. Wie bei dem Fixiergurt 11 ist auch der Ort der lösbaren Anbringung 53 des Kompressionsgurts 13 in seiner Erstreckungsrichtung in einem gewissen Bereich flexibel. Denn die Haken-und-Flausch-Verschlussunterlage 57a auf der Verstärkungsapplikation 9 weist eine gewisse Breite auf, die es zulässt, dass das zweite Ende 51 des Kompressionsgurts 13 an in seiner Erstreckungsrichtung beabstandeten Punkten angebracht werden kann.

Der Kompressionsgurt 13 ist ausgebildet, um von dem Ort der festen Anbringung 55 mit seinem ersten Ende 49 am posterioren Abschnitt 23 des Hauptkörpers 3 entlang der lateralen Seite 19a des Fußsohlenabschnitts 19, um den Fußsohlenabschnitt 19 herum zur medialen Seite 19b des Fußsohlenabschnitts 19 über den Spann- und Ristabschnitt 21 hin zum lateralen Abschnitt 17 und von dort spiralenartig um den Unterschenkel des von dem Hauptkörper 3 umgebenen Fußes bzw. Beins in Richtung abseits des Fußsohlenabschnitts 19 zunächst um den posterioren Abschnitt 23, dann um den medialen Abschnitt 15 bis schließlich hin zum Bereich des lateralen Abschnitts 17 geführt zu werden, der benachbart zu dem oberen Ende 25 des Hauptkörpers 3 ist, um dort auf der Verstärkungsapplikation 9 lösbar damit verbunden zu werden.

Um die Führung des Kompressionsgurts 13 im Bereich des Fußsohlenabschnitts 19 zu vereinfachen und um einen genauen und reproduzierbaren Sitz des Kompressionsgurts 13 in diesem Bereich zu gewährleisten, ist der Kompressionsgurt 13 an dem Fußsohlenabschnitt 19, vorzugsweise auf halber Breite des Fußsohlenabschnitts 19 zwischen lateraler und medialer Seite 19a, 19a benachbart zu dem posterioren Abschnitt 23 eines umschlossenen Fußes, fixiert.

Durch die in Erstreckungsrichtung des Kompressionsgurts 13 flexible bzw. verschiebbare Anbringung 53 des zweiten Endes 51 des Kompressionsgurts 13 an dem Hauptkörper 3 bzw. an der Verstärkungsapplikation 9 kann der von dem Kompressionsgurt 13 auf den Hauptkörper 3, die Verstärkungsapplikation 9 und den Fixiergurt 11 übertragene Druck, der sich im Wesentlichen gleichmäßig auf die darunterliegende zuvor genannte Struktur (Hauptkörper 3, Verstärkungsapplikation 9 und Fixiergurt 11) und somit mittelbar auf das umschlossene Bein bzw. den umschlossenen Fuß überträgt, wunschgemäß angepasst werden.

Außerdem wird durch den zuvor beschriebenen Verlauf des Kompressionsgurts 13 entlang des Hauptkörpers 3 der Druck, der durch ein Spannen des Kompressionsgurtes 13 aufgebracht wird, in gleichmäßiger Weise auf die darunter liegende Struktur (Hauptkörper 3, Verstärkungsapplikation 9 und Fixiergurt 11) verteilt, wobei keine für den Benutzer unangenehmen oder orthopädisch nachteiligen Druckspitzen auftreten. Durch den Druck, den der Kompressionsgurt 13 ausübt, übernimmt dieser eine ähnliche Wirkung wie die einer Bandage und sorgt dafür, dass die darunter angeordneten Schienenelemente 5, 7 möglichst eng und starr an den Unterschenkelknochen, -sehnen und - bändern anliegen und diese schienen.

Die Sprunggelenksorthese 1 kann wie folgt verwendet werden. Der Öffnungsabschnitt 27 wird zunächst geöffnet, indem die Lasche 29 von dem medialen Abschnitt 15 gelöst wird und der Hauptkörper 3 z.B. mit den Händen aufgeweitet wird. Danach steigt ein Benutzer mit seinem Fuß in den Hauptkörper 3 und schließt anschließend den Öffnungsabschnitt 27 indem er die Lasche 29 an den medialen Abschnitt 15 angelegt und dort lösbar fixiert.

Beim Anlegen der Sprunggelenksorthese 1 sind Fixiergurt 11 und Kompressionsgurt 13 vorzugsweise gelockert, d. h. an ihrem zweiten Ende 37, 51 von dem Hauptkörper 3 bzw. der Verstärkungsapplikation 9 gelöst. Zur Erzielung eines Pronationseffekts bei dem von dem Hauptkörper 3 umschlossenen Fuß bringt der Benutzer den Fixiergurt 11 an seinem zweiten Ende 37 auf Spannung und befestigt diesen schließlich derart lösbar mit seinem zweiten Ende 37 über einen Haken-und-Flausch-Verschluss 43a an dem Hauptkörper 3 bzw. an der Verstärkungsapplikation 9, dass das gewünschte Maß an Pronation erzielt wird, die Au-ßenseite des Fußes also im gewünschten Umfang angehoben wird. Dabei stellt die Verschiebbarkeit des Fixiergurts 11 relativ zum Hauptkörper 3 sicher, dass Letzterer nicht gegenüber dem Fuß des Benutzers verdreht, verzogen bzw. tordiert wird, sondern der Fuß als Ganzes tatsächlich wie gewünscht verkippt wird.

Im Folgenden bringt der Benutzer den Kompressionsgurt 13 an seinem zweiten Ende 51 auf Spannung und stellt das gewünschte Maß an Kompression des Fußes ein, indem er das zweite Ende 51 des Kompressionsgurts 13 lösbar an einer geeigneten Stelle der Haken-und-Flausch-Verschlussunterlage 57a im lateralen Abschnitt 17 des Hauptkörpers 3 an der Verstärkungsapplikation 9 anbringt.

Da das laterale und das mediale Schienenelement 5, 7 in dem vorliegenden Ausführungsbeispiel in der bereits beschriebenen Weise gegenüber den Knöchelabschnitten 31, 33 versetzt sind, wird außerdem ein nach posterior gerichteter Druck auf das Sprungbein ausgeübt.

Durch die vorliegende Sprunggelenksorthese 1 können somit u.a. die folgenden drei Effekte erzielt werden:
- Mit dem Fixiergurt 11 wird eine Pronation oder Supination des von der Orthese gehaltenen Fußes erreicht abhängig davon, ob das erste oder zweite Ende 35, 37 des Fixiergurts lösbar mit dem Hauptkörper 3 verbunden ist.
- Mit den Schienenelementen 5, 7 wird der Unterschenkel bzw. der Knöchel gleichzeitig geschient, wobei durch die Lage der Schienenelemente 5, 7 einem Talusvorschub entgegengewirkt wird.
- Durch den Kompressionsgurt 13 wird schließlich der gesamte, durch den Hauptkörper 3 umschlossene Bereich des Sprunggelenks eines Beins vom Unterschenkel bis zum Mittelfuß komprimiert.

## Patentansprüche

1. Sprunggelenksorthese (1)
mit einem flexiblen Hauptkörper (3), der ausgebildet ist, um ein menschliches Bein im Bereich des Sprunggelenks vom Unterschenkel bis zum Mittelfuß zu umgeben,
wobei der Hauptkörper (3) einen medialen Abschnitt (15), einen lateralen Abschnitt (17), einen Fußsohlenabschnitt (19) mit einer lateralen und einer medialen Seite (19a, 19b), und einen Spann- und Ristabschnitt (21) umfasst,
wobei der mediale Abschnitt (15) zur Anlage an die mediale Seite, der laterale Abschnitt (17) zur Anlage an die laterale Seite, der Fußsohlenabschnitt (19) zur Anlage an die Fußsohle und der Spann- und Ristabschnitt (21) zur Anlage an den Spann und/oder den Rist des Fußes vorgesehen ist, und
mit einem um den Hauptkörper (3) wickelbaren Fixiergurt (11), der ein erstes Ende (35) und ein zweites Ende (37) aufweist,
wobei der Fixiergurt (11) mit dem ersten Ende (35) mit dem lateralen Abschnitt (17) des Hauptkörpers (3) verbunden ist und mit dem zweiten Ende (37) mit dem medialen Abschnitt (15) des Hauptkörpers (3) benachbart zu dessen oberem Ende (25) verbunden ist und
wobei der Fixiergurt (11) derart verschiebbar um den Hauptkörper (3) geführt ist, dass der Fixiergurt (11) von dem ersten Ende (35) an dem lateralen Abschnitt (17) ausgehend über den Spann- und Ristabschnitt (21), zur medialen Seite (19b) des Fußsohlenabschnitts (19), über den Fußsohlenabschnitt (19) hinweg zur lateralen Seite (19a) des Fußsohlenabschnitts (19), über den Spann- und Ristabschnitt (21) und bis zum oberen Ende (25) des medialen Abschnitts (15) verläuft,
wobei der Fixiergurt (11) um den Fußsohlenabschnitt (19) herum verläuft, ohne mit dem Hauptkörper (3) verbunden zu sein, sondern an diesem anliegt und verschiebbar dazu ist,
wobei eines aus dem ersten und dem zweiten Ende (35, 37) mit dem Hauptkörper (3) in einer relativ zu dem Hauptkörper fest vorgegebenen Position fest oder lösbar verbunden ist und
wobei das andere aus dem ersten und dem zweiten Ende (35, 37) lösbar mit dem Hauptkörper (3) verbunden ist, **dadurch gekennzeichnet,**
**dass** an dem lateralen Abschnitt (17) des Hauptkörpers (3) ein laterales Schienenelement (5) und an dem medialen Abschnitt (15) des Hauptkörpers (3) ein mediales Schienenelement (7) angebracht ist.

2. Sprunggelenksorthese nach Anspruch 1, **dadurch gekennzeichnet, dass** das eine Ende (35, 37) fest mit dem Hauptkörper (3) verbunden ist.

3. Sprunggelenksorthese nach Anspruch 1, **dadurch gekennzeichnet, dass** das eine Ende (35, 37) in einer aus einer Vielzahl von relativ zu dem Hauptkörper fest vorgegebenen Positionen lösbar mit diesem verbunden ist.

4. Sprunggelenksorthese nach Anspruch 1 oder 3, **dadurch gekennzeichnet, dass** das eine Ende (35, 37) über eine oder mehrere Druckknopfverbindung mit dem Hauptkörper (3) verbunden ist.

5. Sprunggelenksorthese nach einem der Ansprüche 1 bis 4, **dadurch gekennzeichnet, dass** das andere Ende (35, 37) über eine Haken-und-Flausch-Verbindung lösbar mit dem Hauptkörper (3) verbunden ist.

6. Sprunggelenksorthese nach einem der Ansprüche 1 bis 5, **dadurch gekennzeichnet, dass** der Hauptkörper (3) entlang des Verlaufs des Fixiergurts (11) Führungslaschen (47) zur verschiebbaren Führung des Fixiergurts (11) aufweist.

7. Sprunggelenksorthese nach einem der vorherigen Ansprüche, **dadurch gekennzeichnet, dass** an dem Hauptkörper (3) eine Verstärkungsapplikation (9) vorgesehen ist, dass die Verstärkungsapplikation (9) zusammenhängend ausgebildet ist und dass sich die Verstärkungsapplikation (9) von dem oberen Ende (25) des lateralen Abschnitts (17) über diesen, über den Fußsohlenabschnitt (19) und über den medialen Abschnitt (15) bis zu dessen oberem Ende (25) erstreckt.

8. Sprunggelenksorthese nach Anspruch 7, **dadurch gekennzeichnet, dass** das Material der Verstärkungsapplikation (9) in dessen Verlaufsrichtung vom oberen Ende (25) des lateralen Abschnitts (17) zum oberen Ende (25) des medialen Abschnitts (15) nicht-dehnbar ausgebildet ist.

9. Sprunggelenksorthese nach Anspruch 1 oder 7, **dadurch gekennzeichnet, dass** das laterale und das mediale Schienenelement (5, 7) zwischen dem Hauptkörper (3) und einer Verstärkungsapplikation (9) angebracht sind.

10. Sprunggelenksorthese nach einem der vorherigen Ansprüche, **dadurch gekennzeichnet, dass** der Hauptkörper (3) einen posterioren Abschnitt (23) aufweist, der vorgesehen ist, an der posterioren Seite des Fußes anzuliegen, einen lateralen Knöchelabschnitt (31) aufweist, der vorgesehen ist, auf der lateralen Seite des Fußes an dem Knöchel anzuliegen und einen medialen Knöchelabschnitt (33) aufweist, der vorgesehen ist, auf der medialen Seite des Fußes an dem Knöchel anzuliegen,
dass das laterale Schienenelement (5) an dem lateralen Abschnitt (17) des Hauptkörpers (3) derart vom posterioren Abschnitt (23) weg versetzt verläuft, dass das laterale Schienenelement (5) gegenüber dem lateralen Knöchelabschnitt (31) in Richtung zum Spann- und Ristabschnitt (21) versetzt verläuft und
dass das mediale Schienenelement (7) an dem medialen Abschnitt (15) des Hauptkörpers (3) derart zum posterioren Abschnitt (23) hin versetzt angeordnet ist, dass das mediale Schienenelement (7) gegenüber dem medialen Knöchelabschnitt (33) in Richtung zum posterioren Abschnitt (23) versetzt verläuft.

11. Sprunggelenksorthese nach einem der vorherigen Ansprüche, **dadurch gekennzeichnet, dass** ein Kompressionsgurt (13) aus einem elastischen Material vorgesehen ist, der ein erstes und ein zweites Ende (49, 51) aufweist,
dass der Kompressionsgurt (13) um den Hauptkörper (3), den Fixiergurt (11) und die Verstärkungsapplikation (9) gewickelt ist, und
dass das erste Ende (49) des Kompressionsgurts (13) fest und das zweite Ende (51) des Kompressionsgurts (13) lösbar mit dem Hauptkörper (3), dem Fixiergurt (11) oder der Verstärkungsapplikation (9) verbunden ist.

12. Sprunggelenksorthese nach Anspruch 11, **dadurch gekennzeichnet, dass** das erste Ende (49) des Kompressionsgurtes (13) fest an dem posterioren Abschnitt (23) angebracht ist,
dass der Kompressionsgurt (13) ausgebildet ist, vom posterioren Abschnitt (23) über den lateralen Abschnitt (17) zur lateralen Seite (19a) des Fußsohlenabschnitts (19), über den Fußsohlenabschnitt (19) zu dessen medialer Seite (19b), über den medialen Abschnitt (15) und den Spann- und Ristabschnitt (21) zum lateralen Abschnitt (17), über den lateralen Abschnitt (17), den posterioren Abschnitt (23) und über den medialen Abschnitt (15) nach oben zu dem Bereich des lateralen Abschnitts (17) zu verlaufen, der benachbart zum oberen Ende (25) des lateralen Abschnitts (17) ist, und
dass das zweite Ende (51) des Kompressionsgurtes (13) mit dem Bereich des lateralen Abschnitts (17) lösbar verbunden ist, der benachbart zum oberen Ende (25) des lateralen Abschnitts (17) ist.

13. Sprunggelenksorthese nach Anspruch 12, **dadurch gekennzeichnet, dass** der Kompressionsgurt (13) an dem Fußsohlenabschnitt (19) fixiert ist.

## Claims

1. The ankle brace (1), comprising:
a flexible main body (3) adapted to surround a human leg in the area of the ankle from the lower leg to the midfoot,
wherein the main body (3) comprises a medial section (15), a lateral section (17), a sole section (19) having a lateral and medial side (19a, 19b), and an arch and instep section (21),
wherein the medial section (15) is provided to contact the medial side, the lateral section (17) is provided to contact the lateral side, the sole section (19) is provided to contact the sole, and the arch and instep section (21) is provided to contact the arch and/or instep of the foot, and
including a fixing belt (11) having a first end (35) and a second end (37) for being wound around the main body (3),
wherein the fixing belt (11) is connected to the first end (35) with the lateral section (17) of the main body (3) and to the second end (37) with the medial section (15) of the main body (3) adjacent to its top end (25) and
wherein the fixing belt (11) is movably guided around the main body (3) such that the fixing belt (11) extends from the first end (35) at the lateral section (17) from the arch and instep section (21) to the medial side (19b) of the sole section (19) across the sole section (19) to the lateral side (19a) of the sole section (19) across the arch and instep section (21) and up to the top end (25) of the medial section (15),
wherein the fixing belt (11) proceeds around the sole section (19) without being connected to the main body (3), rather the fixing belt (11) only lies against the main body (3) and is shiftable in relation thereto,
wherein one of the first and second end (35, 37) is connected to the main body (3) in a predetermined fixed or releasable position relative to the main body and
wherein the other of the first and second end (35, 37) is releasably connected to the main body (3), **characterized in that**
a lateral splint element (5) is fixed to the lateral section (17) of the main body (3) and a medial splint element (7) is fixed to the medial section (15) of the main body (3).

2. The ankle brace according to claim 1, **characterized in that** the one end (35, 37) is fixedly connected to the main body (3).

3. The ankle brace according to claim 1, **characterized in that** the one end (35, 37) is releasably connected to the main body in a plurality of fixed positions relative to the main body.

4. The ankle brace according to claim 1 or claim 3, **characterized in that** the one end (35, 37) is connected to the main body (3) by one or more pushbutton connections.

5. The ankle brace according to one of claims 1 to 4 **characterized in that** the other end (35, 37) is releasably connected to the main body (3) by a hook-and-loop connection.

6. The ankle brace according to one of claims 1 to 5, **characterized in that** the main body (3) includes guide tabs (47) positioned along the path of the fixing belt (11) to moveably guide the fixing belt (11).

7. The ankle brace according to one of the preceding claims, **characterized in that** a reinforcement application (9) is provided on the main body (3), that the reinforcement application (9) is provided contiguously and that the reinforcement application (9) extends from the top end (25) of and across the lateral section (17) across the sole section (19) and across the medial section (15) up to its top end (25).

8. The ankle brace according to claim 7, **characterized in that** the material of the reinforcement application (9) is adapted to be non-stretchable in its path from the top end (25) of the lateral section (17) to the top end (25) of the medial section (15).

9. The ankle brace according to claim 1 or 7, **characterized in that** the lateral and medial splint elements (5, 7) are affixed between the main body (3) and a reinforcement application (9).

10. The ankle brace according to one of the preceding claims, **characterized in that** the main body (3) includes a posterior section (23) adapted to lie on the posterior side of the foot, a lateral ankle section (31) positioned on the lateral side of the foot to lie on the ankle, and a medial ankle section (33) positioned on the medial side of the foot to lie on the ankle,
that the lateral splint element (5) on the lateral section (17) of the main body (3) extends in an offset direction away from the posterior section (23) such that the lateral splint element (5) extends across from the lateral ankle section (31) toward the arch and instep section (21) in an offset direction and
that the medial splint element (7) on the medial section (15) of the main body (3) is arranged so as to be offset with respect to the posterior section (23) such that the medial splint element (7) extends across from the medial ankle section (33) toward the posterior section (23) in an offset direction.

11. The ankle brace according to one of the preceding claims, **characterized in that** a compression belt (13) of an elastic material is provided that has a first and a second end (49, 51),
that the compression belt (13) is wound about the main body (3), the fixing belt (11) and the reinforcement application, and
that the first end (49) of the compression belt (13) is fixedly attached to the main body (3), the fixing belt (11) or the reinforcement application (9); and the second end (51) of the compression belt (13) is releasably attached to the main body (3), the fixing belt (11) or the reinforcement application (9).

12. The ankle brace according to claim 11, **characterized in that** the first end (49) of the compression belt (13) is fixedly attached to the posterior section (23),
that the compression belt (13) is adapted to extend from the posterior section (23) across the lateral section (17) to the lateral side (19a) of the sole section (19), across the sole section (19) to its medial side (19b), and across the medial section (15) and the arch and instep section (21) to the lateral section (17), across the lateral section (17), the posterior section (23) and medial section (15), upward to the area of the lateral section (17) that adjoins the top end (25) of the lateral section (17), and
that the second end (51) of the compression belt (13) is adapted to be releasably connected to the area of the lateral section (17) that adjoins the top end (25) of the lateral section (17).

13. The ankle brace according to claim 12, **characterized in that** the compression belt (13) is affixed to the sole section (19).

## Revendications

1. Orthèse d'articulation de cheville (1)
avec un corps principal flexible (3) conçu pour entourer une jambe humaine au niveau de l'articulation de la cheville de la jambe jusqu'au milieu du pied,
le corps principal (3) comprenant une portion médiale (15), une portion latérale (17), une portion de plante de pied (19) avec un côté latéral et un côté médial (19a, 19b) et une portion de cou-de-pied (21),
la portion médiale (15) étant conçue pour un appui contre le côté médial, la portion latérale (17) pour un appui contre le côté latéral, la portion de plante de pied (19) pour un appui contre la plante de pied et la portion de cou-de-pied (21) pour un appui contre le cou-de-pied, et
avec une sangle de fixation (11) pouvant être enroulée autour du corps principal (3), qui comprend une première extrémité (35) et une deuxième extrémité (37),
la sangle de fixation (11) étant relié avec la première extrémité (35) avec la portion latérale (17) du corps principal (3) et étant reliée avec la deuxième extrémité (37) avec la portion médiale (15) du corps principal (3), à proximité de son extrémité supérieure (25) et
la sangle de fixation (11) étant ajustable autour du corps principal (3) de façon à ce que la sangle de fixation (11) s'étende à partir de la première extrémité (35) sur la portion latérale (17), passe sur la portion de cou-de-pied (21), vers le côté médial (19b) de la portion de plante de pied (19), passe sur la portion de plante de pied (19) vers le côté latéral (19a) de la portion de plante de pied (19), remonte la portion de cou-de-pied (21) et jusqu'à l'extrémité supérieure (25) de la portion médiale (15),
la sangle de fixation (11) s'étendant autour de la portion de plante de pied (19) sans être reliée avec le corps principal (3), mais s'appuie contre celui-ci et est coulissant par rapport à celui-ci,
l'une parmi la première ou la deuxième extrémité (35, 37) étant reliée de manière fixe ou amovible avec le corps principal (3) dans une position prédéterminée par rapport au corps principal, et
l'autre parmi la première ou la deuxième extrémité (35, 37) étant reliée de manière amovible avec le corps principal (3),
**caractérisée en ce que**
au niveau de la portion latérale (17) du corps principal (3), se trouve un élément de rail latéral (5) et, au niveau de la portion médiale (15) du corps principal (3), se trouve un élément de rail (7).

2. Orthèse d'articulation de cheville selon la revendication 1, **caractérisée en ce qu'**une extrémité (35, 37) est reliée de manière fixe avec le corps principal (3).

3. Orthèse d'articulation de cheville selon la revendication 1, **caractérisée en ce qu'**une extrémité (35, 37) est reliée de manière amovible avec le corps principal, dans une parmi une pluralité de positions prédéterminées par rapport à celui-ci.

4. Orthèse d'articulation de cheville selon la revendication 1 ou 3, **caractérisée en ce qu'**une extrémité (35, 37) est reliée avec le corps principal (3) par l'intermédiaire d'une ou plusieurs liaisons par bouton de pression.

5. Orthèse d'articulation de cheville selon l'une des revendications 1 à 4, **caractérisée en ce que** l'autre extrémité (35, 37) est reliée de manière amovible avec le corps principal (3) par l'intermédiaire d'une liaison à crochet et boucle.

6. Orthèse d'articulation de cheville selon l'une des revendications 1 à 5, **caractérisée en ce que** le corps principal (3) comprend, sur la longueur de la sangle de fixation (11), des brides de guidage (47) pour le guidage coulissant de la sangle de fixation (11).

7. Orthèse d'articulation de cheville selon l'une des revendications précédentes, **caractérisée en ce que**, au niveau du corps principal (3), se trouve une application de renforcement (9), **en ce que** l'application de renforcement (9) est conçue de manière cohérente et que l'application de renforcement (9) s'étend de l'extrémité supérieure (25) de la portion latérale (17), en passant par celle-ci, passe par la portion de plante de pied (19) et la portion médiale (15) jusqu'à son extrémité supérieure (25).

8. Orthèse d'articulation de cheville selon la revendication 7, **caractérisée en ce que** le matériau de l'application de renforcement (9) est conçue de manière inextensible dans sa direction d'extension de l'extrémité supérieure (25) de la portion latérale (17) jusqu'à l'extrémité supérieure (25) de la portion médiale (15).

9. Orthèse d'articulation de cheville selon la revendication 1 ou 7, **caractérisée en ce que** l'élément de rail latéral et l'élément de rail médial (5, 7) sont montés entre le corps principal (3) et une application de renforcement (9).

10. Orthèse d'articulation de cheville selon l'une des revendications précédentes, **caractérisée en ce que** le corps principal (3) comprend une portion postérieure (23) conçue pour s'appuyer contre le côté postérieur du pied, comprend une portion de cheville latérale (31) conçue pour s'appuyer sur le côté latéral du pied contre la cheville et comprend une portion de cheville médiale (33) conçue pour s'appuyer sur le côté médial du pied contre la cheville,
**en ce que** l'élément de rail latéral (5) s'étend au niveau de la portion latérale (17) du corps principal (3), de manière décalée par rapport à la portion postérieure (23), de façon à ce que l'élément de rail latéral (5) s'étende de manière décalée par rapport à la portion de cheville latérale (31) en direction de la portion de cou-de-pied (21) et
**en ce que** l'élément de rail latéral (7) est disposé, au niveau de la portion médiale (15) du corps principal (3), de manière décalée par rapport à la portion postérieure (23) de façon à ce que l'élément de rail médial (7) s'étende de manière décalée par rapport à la portion de cheville médiale (33) en direction de la portion postérieure (23).

11. Orthèse d'articulation de cheville selon l'une des revendications précédentes, **caractérisée en ce qu'**une sangle de compression (13), constituée d'un matériau élastique, est prévue, qui comprend une première et une deuxième extrémité (49, 51),
**en ce que** la sangle de compression (13) est enroulée autour du corps principal (3), de la sangle de fixation (11) et de l'application de renforcement (9) et
**en ce que** la première extrémité (49) de la sangle de compression (13) est reliée de manière fixe et la deuxième extrémité (51) de la sangle de compression (13) est reliée de manière amovible avec le corps principal (3), la sangle de fixation (11) ou l'application de renforcement (9).

12. Orthèse d'articulation de cheville selon la revendication 11, **caractérisée en ce que** la première extrémité (49) de la sangle de compression (13) est montée de manière fixe sur la portion postérieure (23),
**en ce que** la sangle de compression (13) est conçue pour s'étendre de la portion postérieure (23), en passant par la portion latérale (17), vers le côté latéral (19a) de la portion de plante de pied (19), en passant sur la portion de plante de pied (19) vers son côté médial (19b), en passant par la portion médiale (15) et la portion de cou-de-pied (21), vers la portion latérale (17), en passant sur la portion latérale (17), la portion postérieure (23) et en passant sur la portion médiale (15) vers le haut en direction de la zone de la portion latérale (17) qui se trouve à proximité de l'extrémité supérieure (25) de la portion latérale (17) et
**en ce que** la deuxième extrémité (51) de la sangle de compression (13) est reliée de manière amovible avec la zone de la portion latérale (17) qui se trouve à proximité de l'extrémité supérieure (25) de la portion latérale (17).

13. Orthèse d'articulation de cheville selon la revendication 12, **caractérisée en ce que** la sangle de compression (13) est fixée à la portion de plante de pied (19).
